# EUROPEAN PATENT SPECIFICATION

(11) **EP 2 842 545 B1**
(45) Date of publication and mention of the grant of the patent: **12.02.2020**
(21) Application number: 13781750.8
(22) Date of filing: 23.04.2013
(51) Int. Cl.: A61K 9/10, A61K 9/06, A61K 47/44, A61P 17/00, A61P 27/02, B82Y 5/00, B82Y 40/00

(54) **METHOD FOR PRODUCING AN AQUEOUS DISPERSION OF DRUG NANOPARTICLES AND USE THEREOF**
VERFAHREN ZUR HERSTELLUNG EINER WÄSSRIGEN DISPERSION AUS ARZNEIMITTELNANOPARTIKELN UND VERWENDUNG DAVON
PROCÉDÉ DE PRODUCTION D'UNE DISPERSION AQUEUSE DE NANOPARTICULES DE MÉDICAMENT ET SON UTILISATION

(30) Priority: 24.04.2012 JP 2012099285
(43) Date of publication of application: 04.03.2015
(73) Proprietor: Osaka University, Suita-shi, Osaka 565-0871 (JP)
(72) Inventor: BABA, Koichi, Suita-shi Osaka 565-0871 (JP); NISHIDA, Kohji, Suita-shi Osaka 565-0871 (JP); HASHIDA, Noriyasu, Suita-shi Osaka 565-0871 (JP)
(74) Representative: Plasseraud IP
(86) International application number: PCT/JP2013/061819
(87) International publication number: WO 2013/161778

(56) References cited:
- WO-A1-2010/053101
- JP-A- 2006 199 589
- JP-A- 2006 249 010
- JP-A- 2010 505 748
- JP-A- 2010 535 728
- US-A1- 2007 071 685
- US-A1- 2009 238 878
- US-A1- 2011 135 741

## Description

### Technical Field

The present invention relates to a method for producing an aqueous dispersion containing drug nanoparticles dispersed therein.

### Background Art

Recent years have seen great attention drawn to drug nanocrystals, which are nanoscale fine particles of a pharmaceutical drug (in particular, a poorly-water-soluble pharmaceutical drug). Pharmaceutical drugs are hoped to, when in the form of nanoscale particles, have improved permeability to cells and/or tissues. Further, even if bulk powder of a pharmaceutical drug is poorly water-soluble, nanocrystallization of such a pharmaceutical drug can remarkably increase its water solubility. There has thus been increasing research on drug nanocrystals as a superior next-generation formulation in the scientific and pharmaceutical formulation fields.

Techniques reported for producing a pharmaceutical drug in the form of nanoparticles (hereinafter referred to as "drug nanoparticles") include [1] a technique of finely reducing the size of particles of bulk powder of a pharmaceutical drug in a top-down manner with use of a homogenizer through laser ablasion, milling and the like (see Patent Literatures 1 and 2), [2] a technique of preparing drug nanocrystals from aggregates of pharmaceutical drug molecules in a bottom-up manner (for example, precipitation, vapor deposition, emulsion, spray-drying, or freezing) (see Non Patent Literatures 1 and 2), and [3] a combination of the above techniques.

### Citation list

Patent Literature 1
   Japanese Patent Application Publication (Translation of PCT Application), *Tokuhyou,* No. 2010-505748 (Publication Date: February 25, 2010)
Patent Literature 2
   Japanese Patent Application Publication, *Tokukai,* No. 2010-031026 (Publication Date: February 12, 2010)
Patent Literature 3
   WO2010/053101 discloses an eye drop which can achieve high intraocular migration of a medical agent contained therein. It also discloses a method for producing the eye drop.

### Patent literature 4

US 2009/238878 discloses a method of preparing micelles by mixing 2 solutions. A dispersion is prepared by emulsification and homogenizing.
Non Patent Literature 1
   Pharm Res. (2011), vol. 28, pp. 2567-2574
Non Patent Literature 2
   Journal of Controlled Release (2008), vol. 128, pp. 178-183

### Summary of Invention

### Technical Problem

Nanocrystallized powder preparations have been commercialized. There are, however, extremely few commercially successful products in the form of liquid preparations containing drug nanocrystals dispersed in water (hereinafter referred to also as "aqueous dispersion preparation"). This is because of a problematic increase in particle diameter which increase is due to crystal growth (hereinafter referred to also as "particle growth") in water and characteristic of drug nanocrystals. In other words, a pharmaceutical drug prepared in the form of nanoparticles forms large (micrometer- to millimeter-scale) crystal aggregates in a liquid preparation. This has made it difficult to provide an aqueous dispersion preparation containing drug nanoparticles dispersed stably therein.

The present invention has been accomplished to solve the above problem. More specifically, the present invention has an object of providing an aqueous composition containing nanoparticles dispersed therein which aqueous composition is usable stably as an aqueous dispersion preparation.

### Solution to Problem

The inventors of the present invention have conducted diligent studies to solve the above problem, and have consequently discovered that with use of drug nanoparticles prepared from a liquid mixture of an organic solvent and water, the liquid mixture containing (i) an active ingredient as a pharmaceutical drug and (ii) an ointment base, it is possible to disperse nanocrystals of the poorly-water-soluble pharmaceutical drug in water and that a nanoparticle aqueous dispersion so prepared exhibits inhibited crystal growth of nanocrystals and retains the original characteristics of the ointment base contained in the nanoparticles (for example, improving the retentivity of a pharmaceutical drug on an affected part, and maintaining long-term controlled release of a pharmaceutical drug). The inventors have thereby completed the present invention.

Specifically, a method according to claim 1, to produce a nanoparticle aqueous dispersion in which nanoparticles including an active ingredient are dispersed in water, includes in particular a step of freeze-drying a frozen sample of a liquid mixture of (i) a first solution including an organic solvent as a solvent thereof and (ii) a second solution including water as a solvent thereof, the liquid mixture containing an active ingredient and an ointment base, and a step of dispersing the freeze-dried sample in water.

With the above arrangement, the method of the present invention allows production of an aqueous dispersion in which nanoparticles including the active ingredient are dispersed uniformly and in which no crystal growth occurs even after an extended time period of storage.

The method of the present invention may further include a step of verifying that no crystal growth has occurred of particles dispersed in the aqueous dispersion. The method of the present invention, which allows production of an aqueous dispersion in which no nanoparticle crystal growth occurs, maintains a uniform dispersion system and consequently forms no precipitation system. The verifying step may thus be a step of verifying that no precipitation has occurred in the aqueous dispersion.

The method of the present invention, as described above, allows production of an aqueous dispersion that exhibits inhibited nanoparticle crystal growth. This means that the method of the present invention can serve as a method for inhibiting growth of nanocrystals of an active ingredient. This is presumably because rapidly freezing and freeze-drying the liquid mixture of an organic solvent and water, the liquid mixture containing an active ingredient and an ointment base, allows the active ingredient to be coated with the ointment base. The method of the present invention allows an active ingredient to be coated successfully with an ointment base and consequently inhibits growth of nanocrystals of the active ingredient. The active ingredient may, however, be coated unevenly. Thus, the method of the present invention may, as necessary, further include a step of selecting, from among a plurality of aqueous dispersions prepared, a dispersion in which no nanoparticle crystal growth has occurred. This step may be a step of selecting, from among a plurality of aqueous dispersions, an aqueous dispersion in which no precipitation has occurred or a step of recovering a suspension fraction (that is, a step of removing a precipitate that has formed).

The method of the present invention allows to produce a composition including the nanoparticle aqueous dispersion. The composition may be an eye drop as an ointment alternative or skin external preparation. The composition, which contains a nanoscale pharmaceutical drug, may serve to allow an active ingredient of a pharmaceutical drug to reach a deep and far part of a tissue, preferably to treat a disease at a deep and far part of the eye, for example.

### Advantageous Effects of Invention

The present invention as claimed in claim 1, inhibits crystal growth of nanocrystals without impairing the characteristics of an ointment base of imparting controlled release and retentivity to an ointment (unguent). The present invention, in addition, allows a poorly-water-soluble pharmaceutical drug to be dispersed successfully in water while preventing formation of a precipitation for an extended period of time.

The method of the present invention allows to produce a pharmaceutical drug to be applied locally on the nanoscale in the form of an aqueous dispersion preparation, and consequently reduces the disadvantages of an ointment (inconvenience and discomfort) that arise from its application to a wide area covering not only an affected part but also its surrounding area.

### Brief Description of Drawings

Fig. 1 shows an electron micrograph of particles obtained in accordance with one embodiment of the method of the present invention.
Fig. 2 shows an electron micrograph of particles in accordance with a conventional technique.
Fig. 3 shows an electron micrograph of particles obtained in accordance with one embodiment of the method of the present invention.
Fig. 4 shows images of the appearance of dispersions each containing particles obtained in accordance with one embodiment of the method of the present invention or a conventional technique.
Fig. 5 shows electron micrographs of nanoparticles before and after a severe test in accordance with one embodiment of the present invention.
Fig. 6 shows the state of nanoparticles (specifically, the results of measuring particle size distributions and zeta potentials) before and after a severe test in accordance with one embodiment of the present invention.
Fig. 7 shows, in accordance with one embodiment of the present invention, the state of particles that depends on whether lanolin is used or not (specifically, electron micrographs and the results of measuring average particle diameters and zeta potentials).
Fig. 8 shows electron micrographs (before a severe test) of nanocrystals for cases involving various ointment bases in accordance with one embodiment of the present invention.
Fig. 9 shows electron micrographs (after a severe test) of nanocrystals for cases involving various ointment bases in accordance with one embodiment of the present invention.
Fig. 10 shows electron micrographs of nanocrystals prepared with use of various pharmaceutical drugs in accordance with one embodiment of the present invention.
Fig. 11 shows a state of fluorescence on the surface of an eyeball after application of fluorochrome nanoparticles in accordance with one embodiment of the present invention.
Fig. 12 shows a state of fluorescence on a skin surface with hair shaved, the state being observed (180 minutes) after fluorochrome nanoparticles are dropped onto the surface
Fig. 13 shows intraocular migration of a medication obtained in accordance with one embodiment of the method of the present invention.
Fig. 14 shows retina structures.

### Description of Embodiments

### [1: Method for Producing Nanoparticle Aqueous Dispersion]

The present invention provides a method for producing a nanoparticle aqueous dispersion. The present invention, in particular, provides a method for producing a drug nanoparticle aqueous dispersion containing a poorly-water-soluble or water-insoluble pharmaceutical drug (or an active ingredient thereof) successfully dispersed therein. The production method includes (i) freezing a liquid mixture of an organic solvent and water, the liquid mixture containing an ointment base and a target active ingredient (for example, a poorly-water-soluble or water-insoluble pharmaceutical drug), (ii) freeze-drying a frozen sample of the liquid mixture, and (iii) redispersing the freeze-dried sample in water. In other words, the production method of the present invention, to produce a nanoparticle aqueous dispersion, includes (i) a step of freeze-drying a frozen sample of a liquid mixture of a first solution containing an organic solvent as a solvent and a second solution containing water as a solvent and (ii) a step of dispersing the freeze-dried sample in water, the liquid mixture containing an active ingredient and an ointment base.

The active ingredient (poorly-water-soluble or water-insoluble pharmaceutical drug) used suitably for the present invention is not particularly limited. Examples of the active ingredient include steroids (for example, dexamethasone and fluorometholone, and triamcinolone, triamcinolone acetonide, betamethasone, hydrocortisone, prednisolone, cortisone, prednisolone acetate, methylprednisolone, methylprednisolone succinate, betamethasone valerate, and cortisol succinate) for use in suspension-type eye drops described below; calpain inhibitors (for example, calpain inhibitor I, calpeptin, calpain inhibitor II, calpain inhibitor III, calpain inhibitor IV, calpain inhibitor IV-2, calpain inhibitor V, calpain inhibitor VI, calpain inhibitor VII, calpain inhibitor X, calpain inhibitor XI, calpain inhibitor XII, calpastatin peptide, EST, PD145305, PD150606, PD151746, Z-Leu-Leu-Tyr-CHN₂, Z-Leu-Tyr-CH₂Cl, Z-Phe-Tyr-CHO, Z-Leu-Leu-CHO, and leupeptin) that inhibit cell apoptosis and that serve as an inhibitor which acts specifically on calpain (calcium-dependent cysteine protease) to function as a neuroprotective drug or the like; nonsteroidal anti-inflammatory drugs (NSAIDs) such as nepafenac, indomethacin, pranoprofen, diclofenac, and bromfenac; antitumor drugs such as paclitaxel, camptothecin, 7-ethyl-10-hydroxy camptothecin, doxorubicin, taxol, mitomycin C, retinoic acid, and thalidomide; antibacterial agents such as erythromycin and ciclosporin A; neovessel restrainers such as fumarin, COX-2 inhibitor, thalidomide, 2-methoxy oestradiol, matrix catabolic enzyme inhibitor marimastat, low-molecular kinase inhibitor, sorafenib, sunitinib, celecoxib, protamine, heparin, cortisone, prednisolone acetate, sulfated polysaccharide, herbimycin A, and fumagillin; antiviral agents such as aciclovir; antihypertensive drug ingredients such as spironolactone; antihistamic agent ingredients such as loratadine; and antilipemic drug ingredients such as clofibrate.

As described above, the aqueous dispersion produced through the method of the present invention exhibits inhibited nanoparticle crystal growth. This is presumably because rapidly freezing and freeze-drying the liquid mixture of the first solution and the second solution allows the active ingredient to be coated with the ointment base. The active ingredient may, however, be coated unevenly, so that the nanoparticle crystal growth may not be inhibited successfully in the dispersion produced. To eliminate such a possibility, the method of the present invention preferably further includes a step of verifying that no nanoparticle crystal growth has occurred, more preferably further including a step of selecting a dispersion in which no nanoparticle crystal growth has occurred.

Non Patent Literatures 1 and 2 each discuss freezing and then freeze-drying a mixture of t-butyl alcohol and water, the mixture containing a pharmaceutical drug and a matrix dissolved therein, and consequently producing nanoparticles of a pharmaceutical drug contained in a matrix. Non Patent Literatures 1 and 2, however, fail to describe or suggest using an ointment base to produce drug nanoparticles. Non Patent Literatures 1 and 2 each discuss using, as a matrix, mannitol to serve as a carrier that facilitates crystallization of nanoparticles during the freeze-drying. Mannitol is, however, not an ointment base and even has nothing in common with an ointment base in terms of structure.

Non Patent Literature 1 discloses that drug nanocrystals, when dispersed in a solvent, easily form an aggregate or dissolve, that the formation of an aggregate can be reduced by adding a surfactant, and that since a surfactant unfortunately dissolves the pharmaceutical drug partially, the addition of a surfactant leads to formation of crystals with sizes that are not optimum. These descriptions indicate that it is difficult for pharmaceutical drug crystal growth to occur in water because of Ostwald ripening or the like and that it is difficult to redisperse drug nanoparticles in water. Non Patent Literatures 1 and 2, however, fail to disclose or suggest any technique for overcoming the above disadvantages.

As discussed above, drug nanoparticles produced through either of the respective methods of Non Patent Literatures 1 and 2 are difficult to redisperse in water. Further, it is impossible to inhibit drug nanoparticle crystal growth in an aqueous dispersion system.

Patent Literature 1 discloses a technique involving (i) an emulsifier contained in an aqueous phase and (ii) a water-insoluble, non-polymeric hydrophobic organic compound contained in an organic phase together with a pharmaceutical drug. The technique uses a high-pressure homogenizer to prepare an emulsion of the aqueous phase and the organic phase, and removes an organic solvent from the emulsion in a vacuum for production of drug nanoparticles. Patent Literature 2 discloses a technique of milling, in a disperse medium, a pharmaceutical drug with a fatty acid ester adsorbed on the surface for production of drug nanoparticles. Patent Literatures 1 and 2, however, fail to describe or suggest using an ointment base to produce drug nanoparticles or performing a freeze-drying step for production of drug nanoparticles. In addition, it will not be easy for persons skilled in the art to combine a technique of which one of the objects is to inhibit crystal growth and another technique with which it is impossible to inhibit crystal growth.

In the aqueous dispersion produced through the method of the present invention, crystal growth of dispersed particles preferably does not occur until the elapse of at least one week or longer, more preferably one month or longer. Further, even in a case where the aqueous dispersion is stored in a high-temperature environment (40°C or higher, 50°C or higher, or 60°C or higher) that is severe for inhibition of crystal growth, crystal growth of dispersed particles preferably does not occur, more preferably does not occur even in a high-temperature environment until the elapse of at least one week or longer, even more preferably one month or longer. The verifying step is thus preferably performed one week or longer after the dispersing step, more preferably one month, two months, three months, six months, twelve months or longer after the dispersing step. In any of these cases, the verifying step may be performed in the above severe environment.

Examples of the organic solvent for use in the first solution include cyclohexane, benzene, 1,4-dioxane, α,α,α-trifluorotoluene, t-butyl alcohol, t-amyl alcohol, ethyl isothiocyanate, 4-xylene, 2-xylene, 2-chloro-α,α,α-trifluorotoluene, 4-chlorotoluene, cyclohexanol, ethanol, acetone, methanol, 1-propanol, 2-propanol, 1-butanol, 2-butanol, 2-methyl-1-propanol, 2-methyl-2-propanol, chloroform, dibromomethane, butyl chloride, dichloromethane, dimethoxymethane, tetrahydrofuran, diethyl ether, ethylene glycol, dimethyl ether, ethylene glycol diethyl ether, diethylene glycol diethyl ether, triethylene glycol dimethyl ether, t-butyl ethyl ether, t-butyl methyl ether, 2-nitroethanol, 2-fluoroethanol, 2,2,2-trifluoro ethanol, 2-methoxyethanol, i-butyl alcohol, 2-ethoxy ethanol, diethylene glycol, 1-, 2-, 3-pentanol, neo-pentyl alcohol, t-pentyl alcohol, diethylene glycol monomethyl ether, diethylene glycol monoethyl ether, cyclohexanol, anisole, benzyl alcohol, phenol, glycerol, dimethylformamide (DMF), dimethylacetamide (DMAC), 1,3-dimethyl-3,4,5,6-tetrahydro-2(1H)-pyrimidinone (DMPU), 1,3-dimethyl-2-imidazolidinone (DMI), 1-methyl-2-pyrrolidone (NMP), formamide, N-methylacetamide, N-methylformamide, acetonitrile, propanol, isopropanol, dimethyl sulfoxide (DMSO), trimethylamine (TMA), ammonium hydroxide, trifluoroacetic acid (TFA), formic acid, butyl chloride, ethylene glycol dimethyl ether, propionitrile, ethyl formate, methyl acetate, ethyl methyl ketone, ethyl acetate, sulfolane, N,N-dimethylpropionamide, tetramethyl urea, nitromethane, nitrobenzene, hexamethylphosphoramide, 2-, 3-, or 4-picoline, pyrrole, pyrrolidine, morpholine, pyridine, piperidine, acetic acid, propionate, pentane, hexane, toluene, cycloheptane, methylcyclohexane, heptane, ethylbenzene, octane, indane, nonane, naphthalene, ethylene glycol dimethyl ether, propylene glycol, glycerol acetate, monothioglycerol, diethanolamine, ethyl lactate, glycerol formal, N-methylpyrrolidone, polyethyleneglycol 400, isopropyl myristate, n-propanol, n-butanol, dimethyl carbonate, methyl ethyl ketone, methyl isobutyl ketone, 1-pentanol, carbon tetrachloride, hexafluoroacetone, chlorobutanol, dimethyl sulfone, butanol, N,N-dimethylformamide, methylene chloride, isopropyl acetate, butyl acetate, propyl acetate, dimethyl acrylamide, lactic acid, glycolic acid, glacial acetic acid, glyceric acid, benzoic acid, carboxy-terminal oligomer of propanoic acid or lactic acid, glycolic acid, tert-butanol, dioxane, ethylene chloride, methyl ethyl ether, methanesulfonic acid, mesityl acid, and HCl. Preferable among these are cyclohexane, benzene, 1,4-dioxane, α,α,α-trifluorotoluene, t-butyl alcohol, t-amyl alcohol, ethyl isothiocyanate, 4-xylene, 2-xylene, 2-chloro-α,α,α-trifluorotoluene, 4-chlorotoluene, cyclohexanol, ethanol, acetone, methanol, 1-propanol, 2-propanol, 1-butanol, 2-butanol, 2-methyl-1-propanol, 2-methyl-2-propanol, dimethyl sulfoxide (DMSO), and 1-methyl-2-pyrrolidone (NMP). More preferably among these are cyclohexane, benzene, 1,4-dioxane, α,α,α-trifluorotoluene, t-butyl alcohol, t-amyl alcohol, ethyl isothiocyanate, 4-xylene, 2-xylene, 2-chloro-α,α,α-trifluorotoluene, 4-chlorotoluene, cyclohexanol, ethanol, 1-propanol, 2-propanol, 1-butanol, 2-butanol, 2-methyl-1-propanol, and 2-methyl-2-propanol. The organic solvent is, however, not particularly limited as long as it can dissolve a target active ingredient. Persons skilled in the art will understand well that in a case involving a pharmaceutical drug that is not dissolved in a single organic solvent or a pharmaceutical drug with low solubility, mixing a first organic solvent as a main component of the first solution with a second organic solvent as a solubilizing agent allows a target pharmaceutical drug to be dissolved successfully. In other words, the first solution of the present invention may contain (i) a single one of the above organic solvents or (ii) a combination of two or more of the above organic solvents.

The second solution may be water or water containing a dispersing agent as necessary. Nanoparticle crystal growth may, depending on the organic solvent used or the mixing ratio between the first solution and the second solution, not be inhibited successfully in a dispersion produced. However, by further performing the above-described step of verifying that no nanoparticle crystal growth has occurred or step of selecting a dispersion in which no nanoparticle crystal growth has occurred, it is possible to produce a nanoparticle aqueous dispersion in which nanoparticle crystal growth is inhibited successfully. In a case where the organic solvent for use in the first solution is t-butyl alcohol, the liquid mixture of the first solution and the second solution preferably has a mixing ratio in the range of 10000:1 to 1:10000, more preferably in the range of 10:1 to 1:10.

The aqueous dispersion, which exhibits no nanoparticle crystal growth, maintains a uniform dispersion system and consequently forms no precipitation system. The verifying step may thus be a step of verifying that no precipitation has occurred in the aqueous dispersion, and the selecting step may be a step of selecting an aqueous dispersion in which no precipitation has occurred or a step of recovering a suspension fraction (that is, a step of removing a precipitate that has formed).

The claimed ointment base include lanolin, vaseline, beeswax, phenol and zinc oxide liniment, cacao butter, witepsol, glycerogelatin, liquid paraffin, hard fat, macrogol, hydrocarbon gel ointment base (for example, a mixture of liquid paraffin and polyethylene, such as Plastibase available from Taisho Toyama Pharmaceutical Co., Ltd.). The ointment base is more preferably lanolin or a derivative thereof. Lanolin, which is mutton tallow produced as a by-product when wool is separated from raw hair of sheep, contains a mixture of an aliphatic alcohol, a cholesterol, a terpene alcohol, and a fatty acid. Lanolin is in the form of a paste at normal temperature. It has a good water-holding property and can be mixed with an amount of water which amount doubles the self weight, but is essentially water-insoluble. Lanolin derivatives include an alcohol (lauric alcohol) and an acid (lauric fatty acid) each produced by hydrolyzing, fractionating, and refining lanolin. Lanolin derivatives further include a product (for example, a metal salt of lauric fatty acid) produced from lanolin through a chemical reaction such as acetylation, alkoxylation, sulfonation, hydrogenation, transesterification, and reduction. Lanolin or a derivative thereof for use in the method of the present invention may be a mixture of lanolin derivatives, most preferably refined lanolin. The ointment base may as necessary further contain, for example, an emulsifier such as polysorbate 80 (tween series), gelatin, triethanolamine, gum Arabic, tragacanth, sodium lauryl sulfate, polyoxyl 40 stearate, sorbitan mono fatty acid ester (span series), and other surfactants.

The method of the present invention includes a step of dissolving the active ingredient and the ointment base in the organic solvent to prepare the first solution and a step of dissolving the dispersing agent in water to prepare the second solution. The method of the present invention includes a step of preparing the liquid mixture of the first solution and the second solution and a step of rapidly freezing the liquid mixture of the first solution and the second solution. The freezing step, which freezes the liquid mixture rapidly, is preferably performed in an environment where the liquid mixture becomes frozen, for example, (i) at a temperature of 0°C or below, (ii) in a refrigerator, a freezer, or a deep freezer, or (iii) in the presence of a cooling solvent, liquid helium, dry ice, or liquid nitrogen. The freezing step is most preferably performed in the presence of liquid nitrogen.

The term "nanoparticle" as used in the present specification intends to mean a nanometer-size particle. The nanoparticles for use in the present invention preferably have an average particle size of approximately 500 nm, but may have a size of less than 220 nm. Nanoparticles of such a particle size can be obtained through filtration with use of a 0.22 µm filter. The nanoparticles for use in the present invention more preferably have an average particle size of approximately 10 to 200 nm, further preferably approximately 20 to 180 nm, even more preferably approximately 25 to 150 nm, most preferably approximately 25 to 100 nm.

The method of the present invention preferably further includes a step of filtering the dispersion obtained through the dispersing step. The filtering step preferably serves to remove, from the aqueous dispersion, particles having sizes greater than the nanoscale, but may alternatively serve as the step of verifying that no nanoparticle crystal growth has occurred.

The method of the present invention, which uses nanoparticles, can increase the specific surface area and thus improve the solubility of a pharmaceutical drug. In a case involving a poorly-water-soluble pharmaceutical drug, the method of the present invention allows such a poorly-water-soluble pharmaceutical drug to be dispersed in water, the dispersion being necessary for administration into a living body.

The active ingredient for use in the method of the present invention may simply be a pharmaceutical drug that is used in situations where the effects of the nanoparticle aqueous dispersion of the present invention are wanted. The active ingredient for use in the present invention is thus not limited to a particular pharmaceutical drug. Further, the active ingredient for use in the present invention can suitably be such a prodrug as is disclosed in WO 2010/053101.

Persons skilled in the art will be able to appropriately select a pharmaceutical drug (active ingredient) in correspondence with the target disease. What organic solvent(s) dissolves the selected pharmaceutical drug is publicly known or can be easily learned. Persons skilled in the art will thus be able to easily select an organic solvent suitable for the pharmaceutical drug, and will also be able to easily select an ointment base dissolvable in that organic solvent. In other words, persons skilled in the art will be able to prepare the first solution with use of the selected pharmaceutical drug without trial and error. Further, persons skilled in the art will be able to prepare the second solution without any trial and error. Persons skilled in the art will, once having read the present specification, easily understand that implementing the present invention with use of the first solution and the second solution both prepared as above allows production of a nanoparticle aqueous dispersion containing a desired pharmaceutical drug.

The method of the present invention for producing a drug nanoparticle aqueous dispersion as claimed, simply needs to include, as described above, at least (i) the step of freeze-drying a frozen sample of the liquid mixture of the first solution and the second solution and (ii) the step of dispersing the freeze-dried sample in water. The present invention therefore covers in its technical scope a method involving a pharmaceutical drug (active ingredient), an ointment base, and/or an organic solvent that are different from those used in the Examples described below.

The present invention, in other words, has an object of providing a method for producing as claimed a drug nanoparticle aqueous dispersion, the method including (i) the step of freeze-drying a frozen sample of the liquid mixture of the first solution and the second solution and (ii) the step of dispersing the freeze-dried sample in water. The present invention thus does not have an object in the individual pharmaceutical drugs (active ingredients), ointment bases, and organic solvents specifically mentioned in the present specification.

### [2: Nanoparticle Aqueous Dispersion]

The present invention also provides a method for producing a nanoparticle aqueous dispersion. The nanoparticle aqueous dispersion includes nanometer-size particles dispersed in water, the particles containing an ointment base with a target active ingredient (poorly-water-soluble or water-insoluble pharmaceutical drug). The ointment base contained in the nanoparticles dispersed in the nanoparticle aqueous dispersion is not particularly limited as long as it is one of the claimed ones.

When nanoscale crystals are dissolved in water, particle growth (that is, crystal growth) of the crystals is repeated because of a phenomenon called Ostwald ripening. The particles present in the solution then form a mass of particles having large diameters (specifically, micrometer-scale or larger diameters), with the result of a precipitation system being formed in the solution. This indicates that nanocrystal aqueous dispersions prepared through conventional techniques are each a heterogeneous system having a wide particle distribution. Such a heterogeneous system is equivalent to a system in which non-nanocrystals are suspended, and fails to enjoy the advantages of nanocrystals.

The nanoparticle aqueous dispersion obtained with the method of the present invention exhibits no nanoparticle crystal growth. This is presumably because the active ingredient is coated partially or entirely with the ointment base. In other words, the nanoparticle aqueous dispersion includes, dispersed therein, nanoparticles containing an active ingredient (poorly-water-soluble or water-insoluble pharmaceutical drug) coated with an ointment base.

The nanoparticles dispersed in the nanoparticle aqueous dispersion do not start crystal growth until the elapse of at least one week or longer, preferably one month or longer, more preferably two months, three months, six months, twelve months or longer even in a case where the nanoparticle aqueous dispersion is stored in a high-temperature environment (40°C or higher, 50°C or higher, or 60°C or higher). In other words, the nanoparticle aqueous dispersion forms no precipitation system until the elapse of at least one week or longer, preferably one month or longer, more preferably two months, three months, six months, twelve months or longer.

The nanoparticles dispersed in the nanoparticle aqueous dispersion preferably have an average particle size of approximately 500 nm, but may have a size of less than 220 nm. Nanoparticles of such a particle size can be obtained through filtration with use of a 0.22 µm filter. The nanoparticles obtained with the method of the present invention more preferably have an average particle size of approximately 10 to 200 nm, further preferably approximately 20 to 180 nm, even more preferably approximately 25 to 150 nm, most preferably approximately 25 to 100 nm.

The nanoparticles obtained with the method of the present invention, which have a particle size within the above numerical range, will allow the pharmaceutical drug, contained in the nanoparticles, to have remarkably higher migration and will remarkably improve the efficiency with which the active ingredient of the pharmaceutical drug reaches deep and far parts of tissues. The nanoparticles obtained with the method of the present invention will, in a case where it is used as an alternative to eye ointments, allow a pharmaceutical drug to have higher permeability to a deep part of the eye. The nanoparticles will thus make it possible to remedy an intractable disease in a deep and far part of the eye (for example, the retina) which disease has been difficult to remedy with use of a pharmaceutical drug. The increased migration of a pharmaceutical drug will allow the maximum pharmacological effect to be produced with the minimum administration of the pharmaceutical drug. Further, since the nanoparticles contain a pharmaceutical drug in the form of crystals, the nanoparticle aqueous dispersion obtained with the method of the present invention provides a high-density preparation containing a 100% close-packed pharmaceutical drug.

### [3: Composition]

The method of the present invention provides a composition containing a nanoparticle aqueous dispersion. The composition has various applications owing to the properties of the nanoparticle aqueous dispersion described above.

### [a] Ointment Alternative

An ointment (or unguent) is a semisolid external preparation including an active ingredient dispersed in a base such as vaseline. An ointment is applied to an affected part for therapy of a skin disease or the like. Examples of the base (referred to also as "ointment base") include a hydrophobic base (oleaginous base) such as vaseline, a hydrophilic base (such as an emulsion base, water-soluble base, and suspension base), a hydrophobic base, a liniment, a pasta, a plaster, a lotion, and a spray. An ointment is, when used, basically applied in the form of cream, gel, lotion or the like. An ointment can adhere to the skin owing to the properties of the ointment base, and allows the active ingredient to remain on the skin for an extended period of time. An ointment is thus used as a skin external preparation, an oral cavity ointment, an eye ointment or the like.

An ointment, which is retained on a diseased part for an extended period of time, is advantageous in long-term, stable controlled release of a pharmaceutical drug. An ointment is, however, not easy to wash away. In a case of an ointment for an eye disease (eye ointment), in particular, applying the cream causes it to be suspended, with the result that the sight is inconveniently blocked. Further, patients have low compliance with such an ointment. In addition, an ointment, which is applied directly to a diseased part, is limited in terms of target diseased parts to the skin, the eye, the oral cavity and the like: A patient cannot easily apply an ointment to a diseased part inside the body. Further, an ointment is applied over a large area of several millimeters squared to several centimeters squared, so that the pharmaceutical drug is frequently wasted as applied to healthy tissues as well as the diseased part. There is also a problem of side effects including an allergic reaction to the base itself.

The nanoparticles dispersed in the nanoparticle aqueous dispersion of the present invention, as described above, contain an active ingredient (poorly-water-soluble or water-insoluble pharmaceutical drug) and an ointment base, the active ingredient being coated partially or entirely with the ointment base. The nanoparticle aqueous dispersion not only exhibits inhibited crystal growth of nanocrystals, but also retains the original characteristics of the ointment base contained in the nanoparticles (for example, improving the retentivity of a pharmaceutical drug on an affected part, and maintaining long-term controlled release of a pharmaceutical drug).

The composition of the present invention can provide drug nanoparticles in an aqueous dispersion (that is, a liquid preparation), and can be administered in forms (for example, oral administration, injection, and instillation) in which conventional ointment preparations have been difficult to administer. The composition of the present invention is, in one embodiment, an ointment alternative; more specifically, a composition of that embodiment is eye drops. Unlike conventional, application-type eye ointments, the composition obtained with the method of the present invention provides a preparation in the form of an aqueous dispersion, and can be administered in droplets. This eliminates the need for thick application of an ointment preparation as with conventional ointment preparations, thereby allowing the patient to avoid unpleasant stickiness and poor sight arising from application of an ointment preparation as with conventional ointment preparations.

Suspension-type eye drops are commercially available in which a poorly-water-soluble pharmaceutical drug is suspended/dispersed in water. A typical example of such a pharmaceutical drug for use in suspension-type eye drops is steroid. Steroid, which is poorly-water-soluble, forms large masses (several micrometers or larger) of particles in water and precipitates at the bottom of the eye drop container. Such suspension-type pharmaceutical drugs have large particle sizes, and thus have low solubility and low migration to the inside of the eye (5% or lower). The composition obtained with the method of the present invention, which contains a nanoscale pharmaceutical drug, allows a pharmaceutical drug to have higher permeability to cells/tissues, and will improve the migration of a pharmaceutical drug to a deep and far part of the eye which part is affected by inflammation or diseased retina at a posterior part of the eyeball to remedy such diseases, which has been difficult with conventional administration of eye drops. Further, the composition obtained with the method of the present invention will allow the maximum pharmacological effect to be produced with the minimum administration of a pharmaceutical drug, and will reduce the risk of a side effect as a result of a reduction in the dosage of the pharmaceutical drug. In addition, the composition allows the patient to avoid discomfort in the use of a pharmaceutical drug and also provides superior pharmacological effects, which will improve the patient's compliance. The composition will, needless to say, provide long-term retentivity and controlled release owing to the ointment base.

The composition obtained with the method of the present invention is, in another embodiment, used as an ointment alternative; more specifically, a composition of that embodiment is a skin external preparation. The composition provides a preparation in the form of nanoscale particles, and allows a pharmaceutical drug to have improved permeability to tissues or cells. The composition can, as the ointment base produces its intended effects, improve the retentivity of a pharmaceutical drug on an affected part and maintain long-term controlled release of a pharmaceutical drug.

### [b] Further Application

The pharmaceutical drug for use in the method of the present invention is mainly a nanoscale, poorly-water-soluble pharmaceutical drug. The composition, as described above, provides a preparation in the form of nanoscale particles, and allows a pharmaceutical drug to have improved permeability to tissues or cells. The composition, which has the above characteristics, provides a dosage form highly effective in a drug delivery system (DDS), which will allow the maximum effect to be produced with the minimum administration of a pharmaceutical drug. Such a dosage form will produce superior pharmacological effects and improve the patient's compliance. The composition obtained with the method of the present invention, in other words, is used in situations that require an active ingredient of a pharmaceutical drug to reach from the location to which the composition has been provided (tissue surface) to a deep and far part of the tissue. The composition is, in a case where it is used as eye drops as described above, used to treat a disease at a deep and far part of the eye (for example, the retina). The composition, which provides a new dosage form as described above, will be used in various medical fields and bring outstanding economic benefits.

### [4: Method for Preventing Nanocrystal Growth]

The present invention further provides a method for preventing nanocrystal growth. Inhibiting crystal growth of drug nanocrystals is a vitally important issue in formulation of nanocrystal preparations of which the competition of development will become fierce in the future. The present invention provides a method for inhibiting crystal growth by using an ointment base to coat drug nanocrystals. Drug nanocrystals to which the present invention has been applied have a new formulation that retains the characteristics of the coated ointment preparation (that is, controlled release and retentivity).

The method of the present invention includes notably (i) rapidly freezing a liquid mixture of an organic solvent and water, the liquid mixture containing an ointment base and a target active ingredient (for example, a poorly-water-soluble or water-insoluble pharmaceutical drug) and (ii) freeze-drying a frozen sample of the liquid mixture. The method, in other words, includes a step of freeze-drying a frozen sample of a liquid mixture of a first solution including an organic solvent as a solvent thereof, the first solution containing an active ingredient and an ointment base, and a second solution including water as a solvent thereof.

While freezing a liquid mixture (containing a pharmaceutical drug) of an organic solvent and water and then freeze-drying a frozen sample of the liquid mixture provides drug nanocrystals, performing such steps through the method of the present invention allows nanoparticle crystal growth to be inhibited. This is presumably because rapidly freezing and freeze-drying the liquid mixture (containing a pharmaceutical drug and an ointment base) of the first solution and the second solution allows the active ingredient to be coated with the ointment base. The present invention can, as described above, prevent nanocrystal growth. See as appropriate the description under "1: Method for Producing Nanoparticle Aqueous Dispersion" for further details of the method described herein.

Nanoparticle crystal growth may not be inhibited successfully depending on the coating accuracy. To eliminate such a possibility, the method of the present invention preferably further includes a step of verifying that no nanoparticle crystal growth has occurred, more preferably further including a step of dispersing the freeze-dried sample in water and/or a step of selecting a dispersion in which no nanoparticle crystal growth has occurred. The aqueous dispersion, which exhibits no nanoparticle crystal growth, maintains a uniform dispersion system and consequently forms no precipitation system. The verifying step may thus be a step of verifying that no precipitation has occurred in the aqueous dispersion, and the selecting step may be a step of selecting, from among a plurality of aqueous dispersions, an aqueous dispersion in which no precipitation has occurred or a step of recovering a suspension fraction (that is, a step of removing a precipitate that has formed).

### [Examples]

### [1. Preparation of Nanoparticle Aqueous Dispersion]

First, dexamethasone (30 mg), lanolin (60 mg) as an ointment base, and polyoxyethylene (200) polyoxypropylene glycol (70) (60 mg) were dissolved in 10 mL of t-butyl alcohol to prepare a solution A (first solution). Further, polyvinylpyrrolidone (20 mg), hydroxy propyl methyl cellulose (3 mg), and polysorbate 80 (5pl) were dissolved in 10 mL of purified water to prepare a solution B (second solution).

The dexamethasone, the lanolin, the t-butyl alcohol, the polyvinylpyrrolidone, and the polysorbate 80 were from Wako Pure Chemical Industries, Ltd. The polyoxyethylene (200) polyoxypropylene glycol (70) was from NOF Corporation. The hydroxy propyl methyl cellulose was from Shin-Etsu Chemical Co., Ltd.

Next, 10 mL of the solution A was put into a beaker and stirred with use of a magnetic stirrer at approximately 1500 rpm. Then, 10 mL of the aqueous solution B (25°C) was quickly injected with use of a syringe into the solution A (45°C) being stirred. The resulting mixed solution was put into a recovery flask and frozen rapidly with liquid nitrogen. A frozen sample of the mixed solution was freeze-dried for approximately 6 hours with use of a freeze-dryer. A proper quantity of purified water (approximately 10 mL) was mixed with the freeze-dried sample of the mixed solution, and the sample was dispersed in the water through a supersonic treatment. The resulting dispersion was filtered through a filter having a pore size of 0.22 µm. This prepared a nanoparticle aqueous dispersion of dexamethasone. Further, a nanoparticle aqueous dispersion of fluorometholone was prepared through a similar procedure.

### [2. Observation of Particles in Aqueous Dispersion]

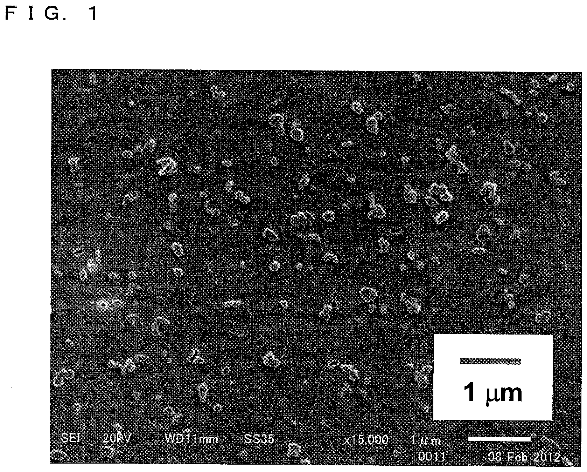

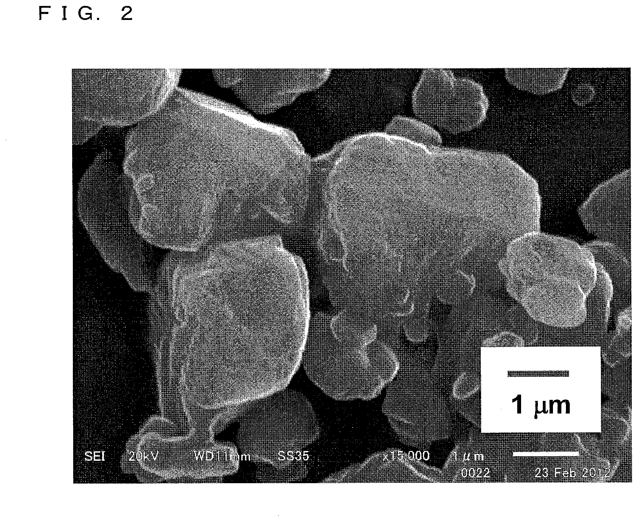

Fig. 1 shows an electron micrograph of dexamethasone nanocrystals in the dexamethasone nanoparticle aqueous dispersion prepared. A dexamethasone aqueous dispersion as a control was prepared through a procedure based on a conventional technique and involving ultrasonic suspension, and dexamethasone nanocrystals in that aqueous dispersion were observed under an electron microscope (see Fig. 2).

The procedure based on a conventional technique was as follows: First, lanolin (60 mg), polyoxyethylene (200) polyoxypropylene glycol (70) (60 mg), polyvinylpyrrolidone (20 mg), hydroxy propyl methyl cellulose (3 mg), and polysorbate 80 (5µl) were mixed with 10 mL of purified water to prepare a liquid mixture. Next, dexamethasone (30 mg) was mixed with the liquid mixture. Then, the resulting mixture was subjected to ultrasonic irradiation for 60 minutes with use of an ultrasonic generator (BRASON 2510). This prepared a dexamethasone aqueous dispersion through ultrasonic suspension.

A 20 µL sample of the nanoparticle dispersion was taken with use of a micropipet. The sample was subjected to reduced-pressure filtration involving an Isopore membrane filter (filter code VMTP available from MILLIPORE) having a pore size of 0.05 µm. This collected a target crystal sample on the Isopore membrane filter. The Isopore membrane filter supporting the crystal sample was attached onto a sample stage for a scanning electron microscope (SEM) (JSM-6510LA available from JEOL) with use of electrically conductive carbon tape. Then, the crystal sample was coated with platinum by sputtering (with use of JFC-1600 available from JEOL) and observed under the SEM.

Fig. 1 shows that nanocrystal groups each having a size of approximately 100 nm were observed. This proved that the method of the present invention allows production of good nanoparticles. Fig. 2 shows that crystal masses each having a size of several micrometers to several tens of micrometers were formed. This proved that a procedure based on a conventional technique fails to produce good nanoscale particles.

The method of the present invention is a bottom-up technique that performs the above rapid freezing for crystallization (aggregation of groups of molecules present in a solution) to prepare nanocrystals (or nanoparticles coated with a base). Conventional production techniques are, in contrast, each a method of providing ultrasonic impact to large-sized masses of particles to crush those masses to smaller sizes in a top-down manner.

Conventional production techniques use an ultrasonic generator, which is limited in its function of finely crushing particles. Specifically, conventional production techniques typically prepare groups of particles having micrometer-scale or larger diameters on average. The method of the present invention, which also uses a similar device for a supersonic treatment during its production process, uses such a device not to crush particles but to provide ultrasonic impact to masses of nanoparticle groups, formed through the freeze-drying step, to soften such masses for redispersion in water.

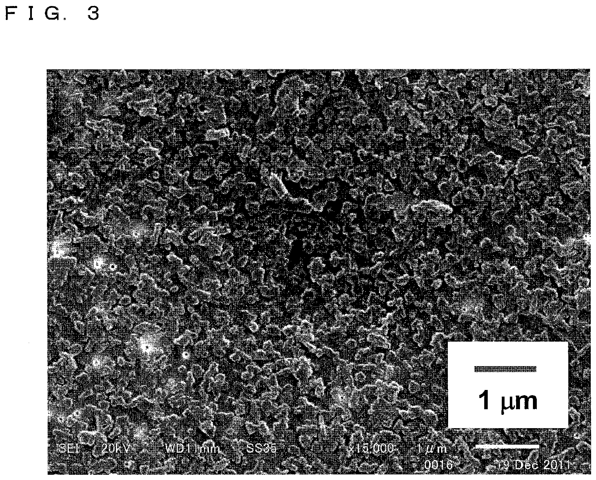

Fig. 3 shows an electron micrograph of fluorometholone nanocrystals in the fluorometholone nanoparticle aqueous dispersion. Similarly to Fig. 1, Fig. 3 shows that nanocrystal groups each having a size of approximately 100 nm were observed. This proved that the method of the present invention allows production of good nanoparticles.

### [3. Appearance of Aqueous Dispersion]

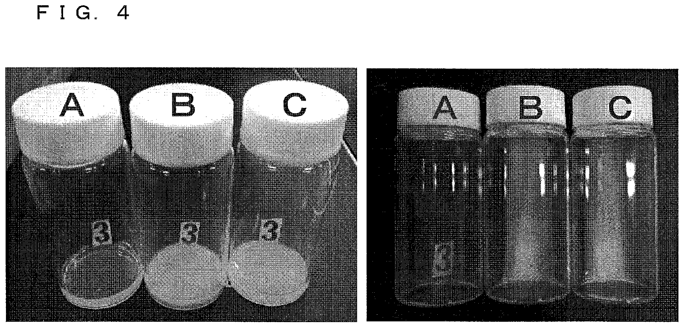

The dexamethasone nanoparticle aqueous dispersion (A) described above, a dexamethasone aqueous dispersion (B) prepared through a conventional technique, and a fluorometholone nanoparticle aqueous dispersion (C) were put into separate vials for observation (see Fig. 4). The vials placed upright (see the left half of Fig. 4) were each laid on its side and seen for a character behind each vial from the opposite side of the vial. The character behind was observed for A and C through the respective dispersions in the vials. This proved that the particles dispersed were small in diameter and that the particles were dispersed uniformly, with the result of reduced light scattering. On the other hand, the character behind was not observed for B through the dispersion in the vial. This proved that the particles dispersed were large in diameter and that the particles were dispersed non-uniformly, with the result of great light scattering.

### [4. Inhibition of Crystal Growth of Nanoparticles]

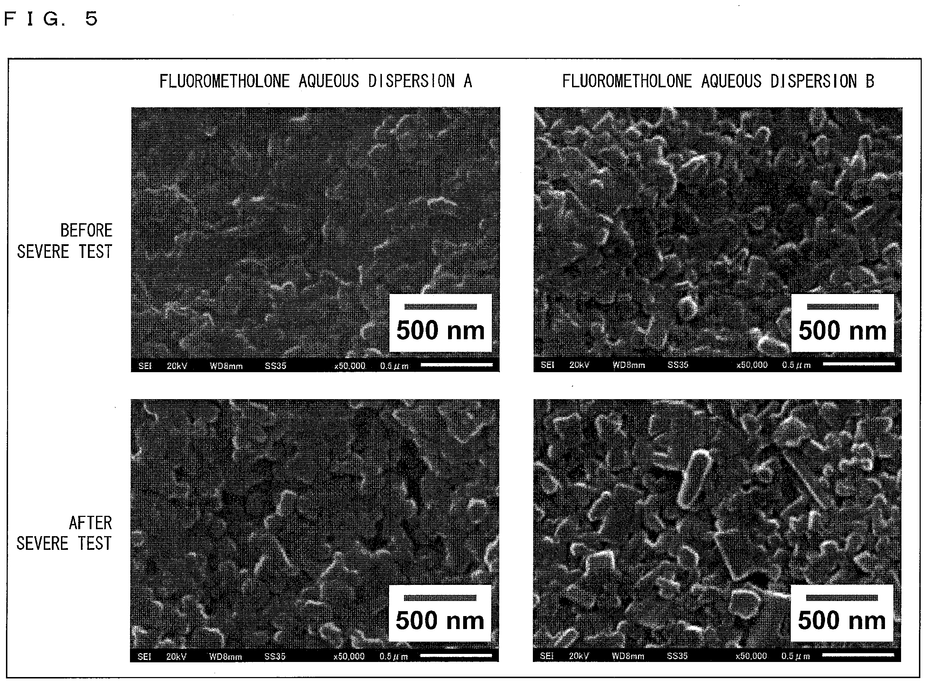

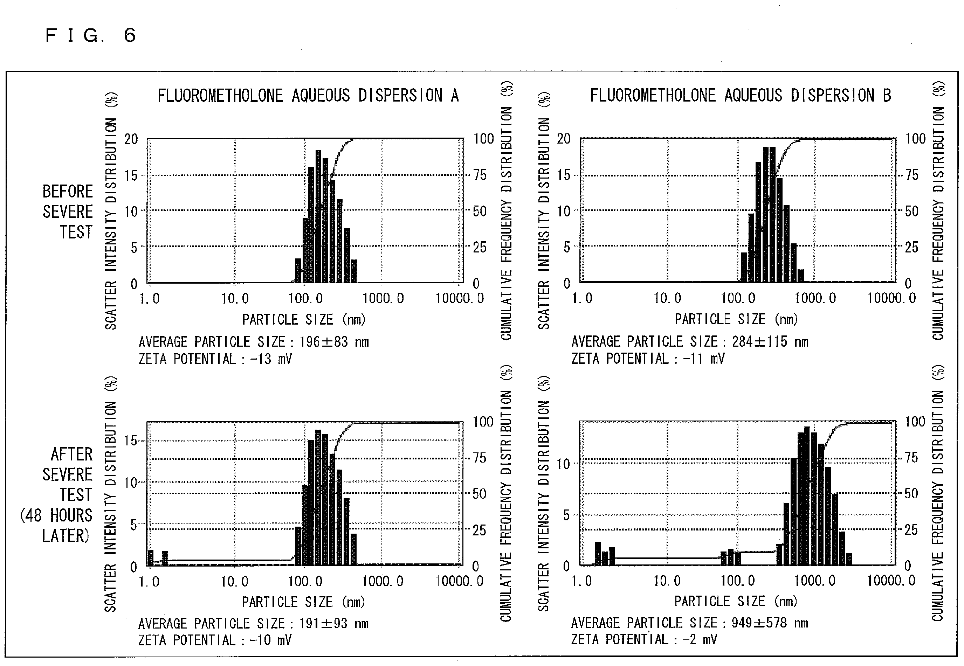

To verify whether crystal growth is inhibited in the nanoparticle aqueous dispersion obtained with the method of the present invention, (i) a fluorometholone aqueous dispersion A containing lanolin (that is, the nanoparticle dispersion of the present invention) and (ii) a fluorometholone aqueous dispersion B containing no lanolin were prepared. A severe test was conducted of leaving the dispersions A and B in a 60°C oil bath for 48 hours. How much crystal growth had occurred through the severe test was examined by (i) electron microscopy and (ii) a particle size distribution measurement and zeta potential measurement both based on dynamic light scattering. The results of the examination showed that the dispersion A had undergone almost no change in the particle diameter, particle size distribution, or zeta potential through the severe test, whereas the dispersion B showed, after the severe test, (i) crystal growth of nanoparticles, (ii) a widened particle size distribution, and (iii) a large decrease in the zeta potential, resulting in unstabilized particle dispersion. Fig. 5 shows electron micrographs. Fig. 6 illustrates particle size distributions and zeta potentials. These results demonstrate the effect of the present invention of inhibiting nanoparticle crystal growth in a nanoparticle dispersion.

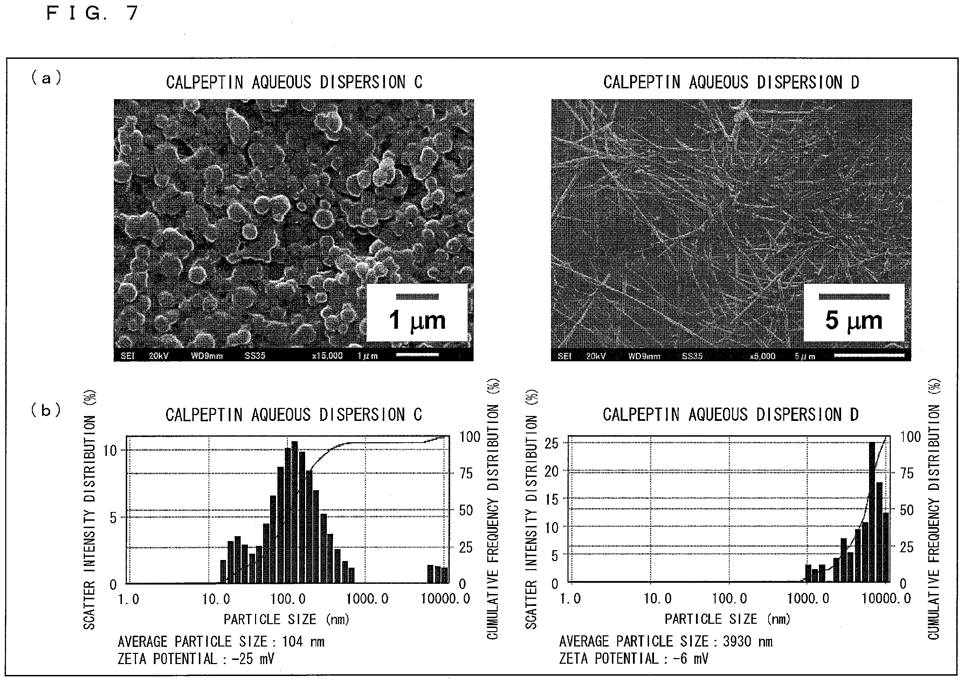

Further, (i) a calpeptin (calpain inhibitor) aqueous dispersion C containing lanolin (that is, the nanoparticle dispersion obtained with the method of the present invention) and (ii) a calpeptin aqueous dispersion D containing no lanolin were prepared. In the calpeptin aqueous dispersion C, in which crystal growth was inhibited, nanoparticles each having a diameter of approximately 100 nm were observed. On the other hand, in the calpeptin aqueous dispersion D, in which crystal growth was not inhibited, fibers of a pharmaceutical drug were formed which had an average size of less than 4 µm. (a) and (b) of Fig. 7 respectively show the results for the dispersions C and D of (i) electron microscopy and (ii) a particle size distribution measurement and zeta potential measurement both based on dynamic light scattering. The zeta potential for the calpeptin aqueous dispersion C was more negative than the zeta potential for the calpeptin aqueous dispersion D. This shows that the nanoparticles in the calpeptin aqueous dispersion C were dispersed with secured stability, demonstrating that the use of an ointment base (lanolin) not only allows crystal growth of a pharmaceutical drug to be inhibited during production of a nanocrystal aqueous dispersion, but also improves the stability with which the nanoparticles are dispersed.

### [5. Aqueous Dispersions Containing Various Ointment Bases]

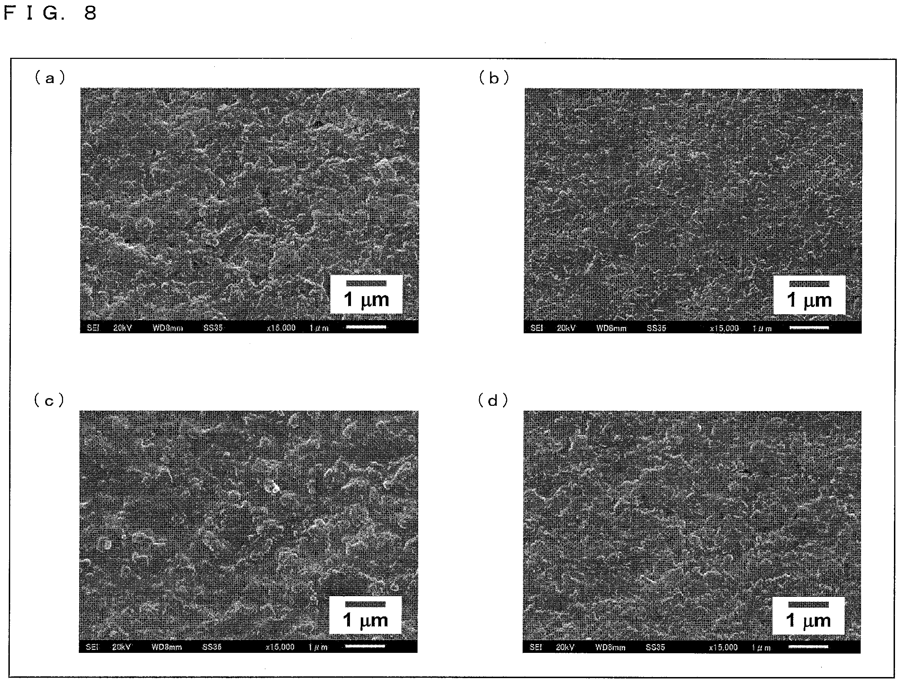

Nanoparticle aqueous dispersions of fluorometholone were prepared through a procedure similar to the procedure for producing a nanoparticle aqueous dispersion containing lanolin as an ointment base, the nanoparticle aqueous dispersions respectively containing (a) vaseline, (b) beeswax, (c) witepsol, and (d) cacao butter as ointment bases. Fig. 8 shows electron micrographs of nanocrystals in the nanoparticle aqueous dispersions prepared. Similarly to the case of Fig. 1, nanocrystal groups each having a size of approximately 200 nm were observed. This proved that the method of the present invention allows production of good nanoparticles. Further, Table 1 shows the results of measuring a particle size distribution and zeta potential, based on dynamic light scattering, for nanocrystals in each nanoparticle aqueous dispersion prepared. The results show that the particles dispersed were small in diameter, that the particles were dispersed uniformly, with the result of reduced light scattering, and that nanoparticles negatively charged on the surface were dispersed stably as the zeta potential measurement indicated.

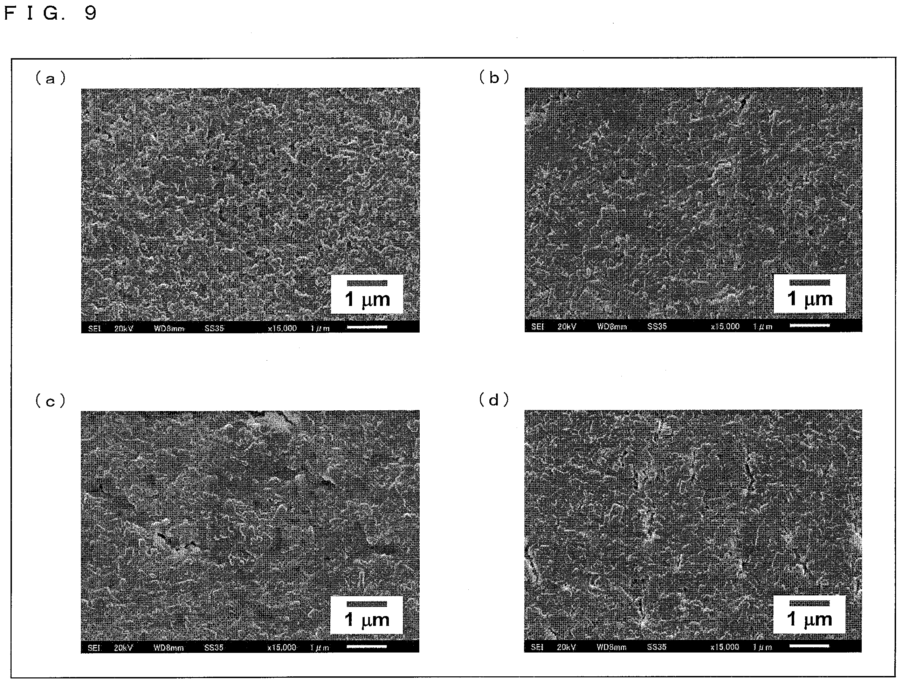

Next, the fluorometholone nanoparticle aqueous dispersions respectively containing (a) vaseline, (b) beeswax, (c) witepsol, and (d) cacao butter as ointment bases were each subjected to the severe test described above (that is, left in a 60°C oil bath for 48 hours). The fluorometholone nanoparticle aqueous dispersions each showed almost no change in the particle diameter or particle size distribution through the severe test. This also demonstrates the effect of the present invention of inhibiting nanoparticle crystal growth in a nanoparticle dispersion even in any of various systems involving different ointment bases. Fig. 9 shows electron micrographs of nanocrystals in the nanoparticle aqueous dispersions prepared. Table 1 shows the results of measuring a particle size distribution and zeta potential, based on dynamic light scattering, for nanocrystals in each nanoparticle aqueous dispersion prepared.

### [Table 1]

**Table 1: Results of Measuring Average Particle Sizes and Zeta Potentials for Nanoparticle Aqueous Dispersions for Cases Involving Various Ointment Bases**

| **Pharmaceutical drugs (ointment used)** | **Before or after severe test** | **Average particle size (nm)** | **Zeta potential (mV)** |
|---|---|---|---|
| Fluorometholone (beeswax) | Before | 194.4 | -8.83 |
| Fluorometholone (witepsol) | Before | 194.8 | -3.75 |
| Fluorometholone (vaseline) | Before | 199.2 | -1.18 |
| Fluorometholone (cacao butter) | Before | 179.1 | -11.79 |
| Fluorometholone (beeswax) | After | 207 | -8.37 |
| Fluorometholone (witepsol) | After | 201.9 | -2.66 |
| Fluorometholone (vaseline) | After | 205.7 | -9.09 |
| Fluorometholone (cacao butter) | After | 200.2 | -11.83 |

### [6. Aqueous Dispersions Containing Various Pharmaceutical Drugs]

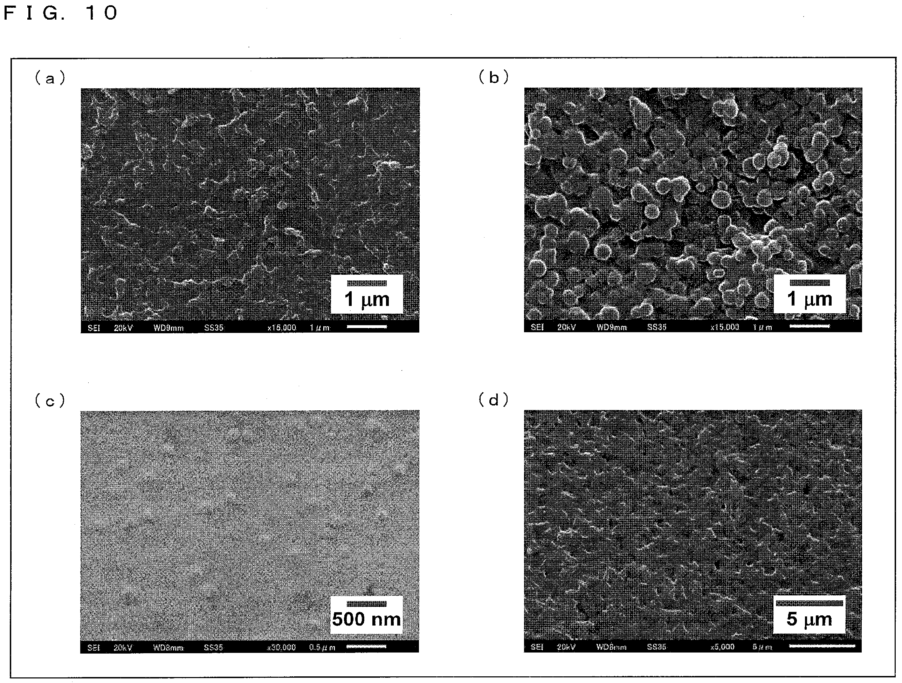

Nanoparticle aqueous dispersions containing respective pharmaceutical drugs other than steroid were prepared through a procedure similar to the procedure for producing a nanoparticle aqueous dispersion of steroid (dexamethasone or fluorometholone). Fig. 10 shows electron micrographs of nanocrystals in the nanoparticle aqueous dispersions prepared. (a) to (d) of Fig. 10 show images of nanocrystals in nanoparticle aqueous dispersions respectively containing calpain inhibitor I, calpeptin, ciclosporin A, and 7-ethyl-10-hydroxy camptothecin as pharmaceutical drugs. Similarly to the case of Fig. 1, nanocrystal groups each having a size of approximately 50 to 400 nm were observed. This proved that the method of the present invention allows production of good nanoparticles. Further, Table 2 shows the results of measuring a particle size distribution and zeta potential, based on dynamic light scattering, for nanocrystals in each of the nanoparticle aqueous dispersions prepared to contain various pharmaceutical drugs. Note that "ALLN" and "SN38" in Table 2 respectively denote calpain inhibitor I and 7-ethyl-10-hydroxy camptothecin. The results show that the particles dispersed were small in diameter, that the particles were dispersed uniformly, with the result of reduced light scattering, and that nanoparticles negatively charged on the surface were dispersed stably as the zeta potential measurement indicated. The results further show that the present invention allows production of nanoparticles of not only a low-molecular compound but also a pharmaceutical drug in the form of a peptide (calpeptin) or oil droplet (clofibrate).

### [Table 2]

**Table 2: Average Particle Sizes and Zeta Potentials for Nanoparticle Aqueous Dispersions for Cases Involving Various Pharmaceutical Drugs**

| **Pharmaceutical drugs** | **Average particle size (nm)** | **Zeta potential (mV)** |
|---|---|---|
| ALLN | 372.2 | -31.38 |
| Calpeptin | 104.9 | -25.36 |
| Nilvadipine | 214.1 | -9.57 |
| Latanoprost | 83.7 | -22.92 |
| Nifedipine | 99.6 | -18.28 |
| SN38 | 232.7 | -15.97 |
| Paclitaxel | 171.9 | -8.53 |
| Ciclosporin | 123.6 | -25.28 |
| Spironolactone | 259.3 | -13.48 |
| Clofibrate | 53.7 | -26.49 |
| Loratadine | 417.6 | -10.73 |
| Erythromycin | 81.8 | -17.65 |
| Retinoic acid | 269.5 | -12.34 |

### [7. Retentivity of Compound Achieved by Nanoparticle Aqueous Dispersion of Present Invention: Part 1]

To verify whether the nanoparticle aqueous dispersion obtained with the method of the present invention allows a compound (nanocrystals) to be retained on a target tissue for an extended period of time, (i) an aqueous dispersion E containing lanolin (that is, the nanoparticle dispersion obtained with the method of the present invention) and (ii) an aqueous dispersion F containing no lanolin were prepared. These aqueous dispersions, each containing fluorescein (CAS 2321-07-5 available from Aldrich) as a fluorochrome, were prepared through the following procedure:
(1) A fluorescein aqueous dispersion E containing lanolin was prepared through the above procedure from (i) a solution A (first solution) prepared by dissolving lanolin (60 mg), Unilube (60 mg), and fluorochrome fluorescein (1 mg) in t-butyl alcohol (10 mL) and (ii) a solution B (second solution) prepared by dissolving PVP (20 mg), Tween 80 (5 µL), and hydroxy propyl methyl cellulose (HPMC) (3 mg).
(2) A fluorescein aqueous dispersion F containing no lanolin was prepared through the above procedure from (i) a solution A (first solution) prepared by dissolving Unilube (60 mg) and fluorochrome fluorescein (1 mg) in t-butyl alcohol (10 mL) and (ii) a solution B (second solution) prepared by dissolving PVP (20 mg), Tween 80 (5 µL), and hydroxy propyl methyl cellulose (HPMC) (3 mg).

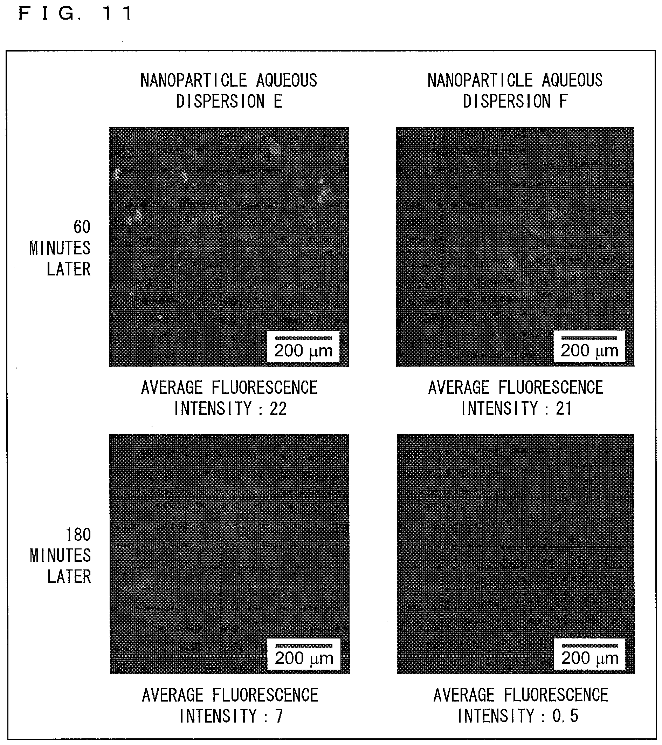

The aqueous dispersions prepared were each applied to a mouse in an amount of 10 µL. After the elapse of a predetermined time period (60 minutes or 180 minutes), the mice were euthanized. Then, the surface of an eyeball of each mouse was examined for fluorescence under a confocal laser scanning microscope. As the result, fluorescence due to a fluorochrome was observed in either case on the surface of an eyeball of the mouse to which the aqueous dispersion E (containing lanolin) was applied (see Fig. 11). In contrast, while fluorescence was observed for the aqueous dispersion F (containing no lanolin) 60 minutes after its application, fluorescence from the surface of an eyeball attenuated over time after the application, with the result that almost no fluorescence was observed 180 minutes after the application (see Fig. 11). This revealed that forming nanocrystals in the nanoparticle dispersion obtained with the method of the present invention improves the retentivity of the compound on a target tissue.

### [8. Retentivity of Compound Achieved by Nanoparticle Aqueous Dispersion of Present Invention: Part 2]

The aqueous dispersion E (that is, the nanoparticle dispersion of the present invention) and the aqueous dispersion F were used to verify whether the nanoparticle aqueous dispersion obtained with the method of the present invention allows a compound (nanocrystals) to be retained for an extended period of time on not only the eyeball but also the skin.

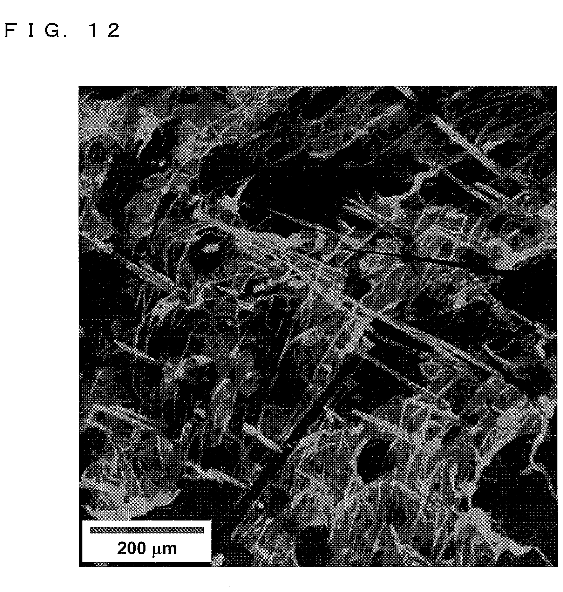

The aqueous dispersions were each dropped in an amount of 50 µL to the skin of a mouse with its hair shaved, and the mice were euthanized after the elapse of 180 minutes. The locations to which the aqueous dispersions were dropped were observed under a confocal laser scanning microscope. As the result, fluorescence due to a fluorochrome was observed at the location to which the aqueous dispersion E (containing lanolin) was dropped (see Fig. 12). In contrast, fluorescence due to a fluorochrome was, in comparison to the case of the aqueous dispersion E, not so clear at the location to which the aqueous dispersion F (containing no lanolin) was dropped. This presumably indicates that the aqueous dispersion F was not retained at the dropped location for an extended period of time and was instead gradually diffused to an area surrounding the dropped location. This revealed that forming nanocrystals in the nanoparticle dispersion obtained with the method of the present invention improves the retentivity of the compound on a target tissue.

Fluorescence was observed in urine of the mouse to which the aqueous dispersion E was dropped, 180 minutes after the drop. This revealed that the nanoparticle aqueous dispersion obtained with the method of the present invention allows controlled percutaneous release of a target compound into the body.

### [9. Effect of Eye Drops Containing Nanoparticle Aqueous Dispersion obtained with the method of Present Invention: Part 1]

Eye drops were prepared from the nanoparticle aqueous dispersion obtained with the method of the present invention, and an anti-inflammatory effect of the eye drops was verified for uveoretinitis, which is a disease of a deep part of the eye. To evaluate the anti-inflammatory effect for uveoretinitis, an experimental autoimmune uveoretinitis (EAU) was selected, which is an animal model of human endogenous uveitis.

In a case of EAU, injecting an S antigen (retina specific antigen) and/or interphotoreceptor retinoid binding protein (IRBP) into a location away from the eye can efficiently induce uveoretinitis with no need for contact with the eye for animals ranging from small animals such as mice and rats to animals close to human beings such as monkeys. The degree of intraocular inflammation can be evaluated objectively and quantitatively on the basis of the clinical scores below with reference to a previous report. A preliminary experiment verified that not less than 95% of individual immunized mice show clinical scores of 2 to 3.

An EAU model animal was prepared with reference to a document (Clin. Exp. Immunol., 1998, 111, 442-9), mice were deeply anesthetized, and a 5 mg/mL peptide solution prepared by sufficiently mixing a complete freund's adjuvant (CFA) containing killed tubercle bacilli with synthetic peptides (N-terminal 15 amino acid of IRBP) was subcutaneously injected into each of the mice in an amount of 200 µL. Normally, uveoretinitis inflammation starts to appear approximately 8 days after the injection, and the inflammation peaks 17 or 18 days after the injection. In view of this, to verify the therapeutic effect of the eye drops obtained with the method of the present invention, the eye drops were applied to the mice three times a day every day over a period of one week from 12 days after the injection to 18 days after the injection. Specifically, (i) a control liquid containing only an eye drop base with no fluorometholone was applied to a control group of three mice, (ii) commercially available eye drops (having a fluorometholone concentration of 0.1 weight%) were applied to a group of two mice, and (iii) the eye drops obtained with the method of the present invention (having a fluorometholone concentration of 0.1 weight%) were applied to a group of two mice.

The eye drop application was stopped one week after its start, and the retina at the fundus of each mouse was observed under a microscope to evaluate the degree of inflammation at the retina site on the basis of clinical scores. Table 3 shows clinical scores indicative of criteria for evaluating an inflammation degree. Table 4 shows the results of the experiments.

**[Table 3]**

| **Scores** | **Clinical score** |
|---|---|
| 0.5 | About one or two minute peripheral or local chorioretinal lesions observed. Mild angitis observed. |
| 1 + | Moderate angitis. Five or fewer local chorioretinal lesions or one or fewer series of chorioretinal lesions observed. |
| 2 + | More than five multiple chorioretinal lesions or inflammatory cell infiltrations observed. Grave angitis over a wide area with cellular infiltration. Five or fewer series of chorioretinal lesions observed. |
| 3 + | Chorioretinal lesions over a wide area, greatly fusogenic chorioretinal lesions, or subretinal neovascularization observed. |
| 4+ | Retinal detachment and/or retinal atrophy over a wide area. |

**[Table 4]**

| **EAU mouse group for clinical trial** | **Clinical score** | |
|---|---|---|
| | **Right eye** | **Left eye** |
| Control liquid | | |
| First mouse | 2+ | 2+ |
| Second mouse | 2+ | 2+ |
| Third mouse | 2+ | 2+ |

| Commercially available eve drops | | |
|---|---|---|
| First mouse | 2+ | 2+ |
| Second mouse | 1+ | 1+ |

| Eve drops of the present invention | | |
|---|---|---|
| First mouse | 1+ | 1+ |
| Second mouse | 0.5 | 0.5 |

For the group of mice to which a commercially available eye drop liquid was applied, two eyes were rated "2+", and the other two eyes were rated "1+". For the group of mice to which the eye drops of the present invention were applied, two eyes were rated "1+", and the other two eyes were rated "0.5". These results show that the group of mice to which the eye drops obtained with the method of the present invention were applied exhibited an anti-inflammatory effect superior to that for the group of mice to which commercially available eye drops were applied or for the group of mice to which a control liquid was applied. The eye drops obtained with the method of the present invention will, as demonstrated above, produce a superior anti-inflammatory effect for uveoretinitis at a posterior part of the eyeball.

### [10. Effect of Eye Drops Containing Nanoparticle Aqueous Dispersion of Present Invention: Part 2]

Eye drops were prepared from the nanoparticle aqueous dispersion obtained with the method of the present invention and applied to rabbits for evaluation of intraocular migration of a medication. Specifically, (i) a control liquid containing only an eye drop base with no fluorometholone was applied to a system (one eye), (ii) commercially available eye drops (having a fluorometholone concentration of 0.1 weight%) were applied to a system (one eye), (iii) the eye drops obtained with the method of the present invention (having a fluorometholone concentration of 0.1 weight%) were applied to a system (one eye), and (iv) no eye drops were applied to a system (one eye).

For each system involving eye drops, the rabbit was anesthetized 30 minutes after the eye drop application, and the cornea was needled with a syringe to sample some hydatoid. The concentration of a medication having migrated into the eye was measured by high-performance liquid chromatography (HPLC) with reference to a document (Atarashii Ganka [Journal of the Eye], 7, 1051-53, 1990). First, 1.5 mL of dichloromethane was added to 0.2 mL of each of various hydatoids sampled, and the resulting mixture was shaken for 10 minutes. After the shake, 1 mL of a dichloromethane layer was separated and evaporated to dry it in a water bath at approximately 45°C. The dichloromethane layer separated was left to stand overnight at room temperature, and the residue was dissolved in 40 µL of methanol, to prepare a sample for HPLC. This operation involved a test tube, a pipet and the like that were sufficiently washed.

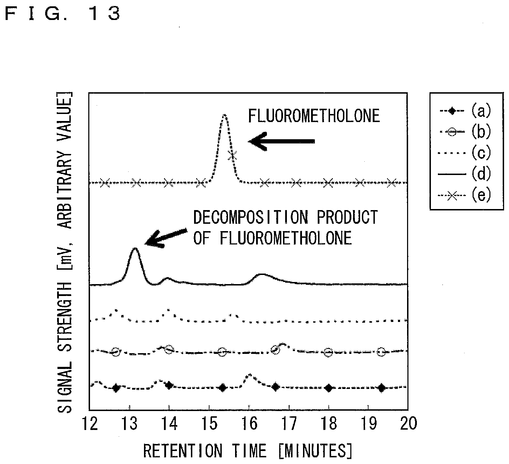

Fluorometholone is known to be mostly decomposed into dihydro fluorometholone after its application and appear in hydatoid. Dihydro fluorometholone is detected in HPLC at an earlier point in retention time than fluorometholone (Atarashii Ganka [Journal of the Eye], 7, 1051-53, 1990). Fig. 13 shows the results of HPLC. For the system to which the eye drops of the present invention were applied (indicated by (d) in Fig. 13), a specific peak was expressed at an earlier point in retention time than fluorometholone (indicated by (e)). This peak was never seen in untreated hydatoid (indicated by (a)), and was little detected in hydatoid from the system to which a control liquid was applied (indicated by (b)) or from the system to which commercially available eye drops were used (indicated by (c)). These results presumably indicate that dihydro fluorometholone (decomposition product of fluorometholone) was contained in a large amount in hydatoid from the system to which the eye drops obtained with the method of the present invention were used (indicated by (d)). A verification experiment was conducted involving a reference sample to confirm that the above peak was a peak for dihydro fluorometholone (the results of which experiment is not shown). The concentration of fluorometholone having migrated into the hydatoid was found on the basis of the above peak to be approximately 235 ng/mL. The intraocular migration was approximately 7 to 8 times higher than reported in the document (Atarashii Ganka [Journal of the Eye], 7, 1051-53, 1990), that is, than approximately 30 ng/mL in a system to which commercially available eye drops were used. This demonstrated that the eye drops obtained with the method of the present invention are higher in tissue permeability (intraocular migration) than conventional eye drops.

### [11. Effect of Eye Drops Containing Nanoparticle Aqueous Dispersion obtained with the method of Present Invention: Part 3]

Eye drops were prepared from the nanoparticle aqueous dispersion obtained with the method of the present invention, and a therapeutic effect of the eye drops was verified for retinal degeneration diseases. Calpain inhibitors (calpain inhibitor I and calpeptin) were used as pharmaceutical drugs. Application of the eye drops twice a day was started to a mouse on the fifth day from birth, the mouse being a mouse (C3H/HeNCrlCrlj) congenitally exhibiting retinal degeneration. As controls, (i) a suspension-type calpain inhibitor eye drop liquid containing coarse particles (that is, not nanoparticles) and (ii) an eye drop liquid containing only a base with no calpain inhibitor were used. The C3H/HeNCrlCrlj mouse has gene mutation at phosphodiesterase (PDE) 6B. Retinal degeneration starts immediately after birth, and by the second week after birth, many retinal pigment epithelial cells and visual cells have undergone apoptosis. The C3H/HeNCrlCrlj mouse is presumed to be a model animal for retinitis pigmentosa in human beings. In evaluation of visual cell survival, evaluating (i) the cell density of an external granular layer in which the nucleus of a visual cell is present or (ii) the state of the layer structure means evaluating the state of neuroprotection. This has been reported as an established method.

The mouse was euthanized 9 days after the start of the eye drop application, and its eyeballs were removed. Then, the eyeball tissues were chemically fixed with use of formalin. The fixed eyeball tissues were immersed in an OCT compound to freeze and harden the tissues, and then shaped into a thin film with use of a cryostat. The resulting slice was subjected to hematoxylin-eosin staining (HE staining), and then observed under an optical microscope for the retina structure.

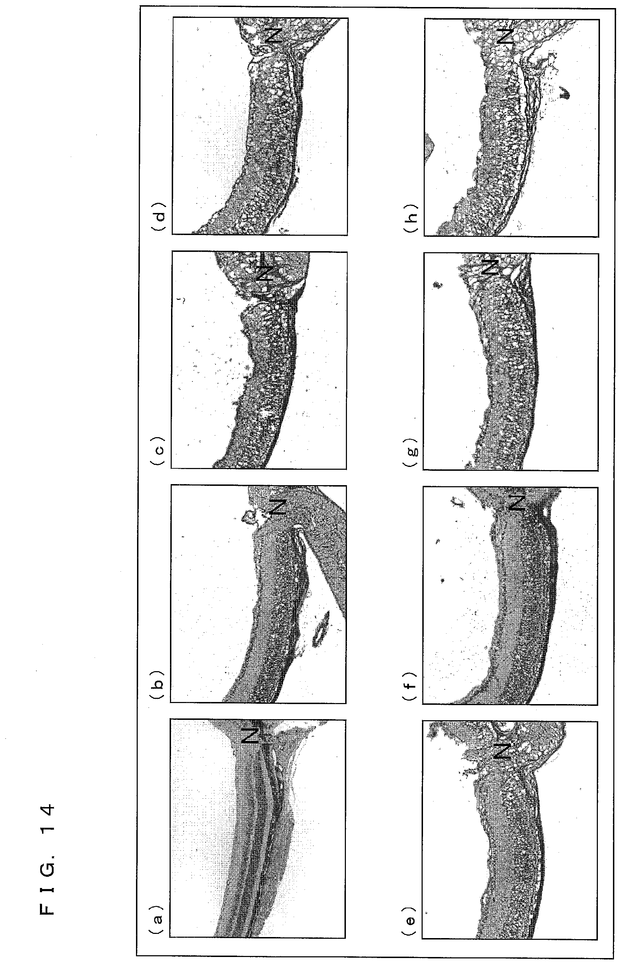

Fig. 14 shows the results of the observation. In Fig. 14, (a) shows the retina of a normal mouse, whereas (b) to (h) each show the retina of a mouse with retinal degeneration. The following solutions were applied to the mice: (b) calpain inhibitor I nanoparticle aqueous dispersion, (c) calpain inhibitor I suspension, (d) no solution applied (control), (e) base only (control), (f) calpeptin nanoparticle aqueous dispersion, (g) calpeptin suspension, and (h) no solution applied (control). Fig. 14 also shows the letter "N" to indicate the optic nerve head.

The normal mouse with no retinal degeneration had a retina with several tens of retained external granular layers (see (a) of Fig. 14), whereas both (i) the control group of mice with retinal degeneration to which mice no eye drops had been applied (see (d) and (h) of Fig. 14) and (ii) the group to which only a base was applied (see (e) of Fig. 14) each had significantly decreased external granular layers, decreased cell density over the entire retina layers, and a large number of vacuoles. The group to which the calpain inhibitor I nanoparticle aqueous dispersion had been applied (see (b) of Fig. 14) had no vacuoles, high cell density, and external granular layers having a cell count and density greater than those of the control group to which no eye drops were applied (see (d) of Fig. 14). Similarly, the group to which the calpeptin nanoparticle aqueous dispersion had been applied (see (f) of Fig. 14) had inhibited apoptosis, seemed to have intraretinal layers with the same level as that for a normal mouse (see (a) of Fig. 14), and had external granular layers with high cell density and thick cell-layer structure. The groups to which not nanoparticle aqueous dispersions but suspensions were applied (see (c) and (g) of Fig. 14) each had a cell density slightly higher than the control group to which no eye drops were applied, but had a large number of vacuoles and uninhibited apoptosis. In comparison of the groups to which nanoparticle aqueous dispersions were applied (see (b) and (f) of Fig. 14) with the groups to which suspensions were applied (see (c) and (g) of Fig. 14), the former groups each clearly had an overwhelmingly higher cell density, no vacuoles, and inhibited apoptosis. This demonstrates that the eye drops obtained with the method of the present invention have a therapeutic effect for retinal degeneration diseases.

It had been believed extremely difficult to use eye drops to remedy such diseases at a deep and far part of the eye as glaucoma (which is a neurodegenerative disease) and retina intractable diseases (for example, age-related macular degeneration, diabetic retinopathy, and retinitis pigmentosa). The eye drops obtained with the method of the present invention had demonstrated its therapeutic effect for retinal degeneration diseases, which strongly suggests that the eye drops obtained with the method of the present invention make it possible to remedy diseases at a deep and far part of the eye. The method of the present invention, which makes it possible to remedy diseases at a deep and far part of the eye in an easy mode (that is, application of eye drops), contributes greatly to eye disease therapy.

### Industrial Applicability

The present invention can provide a technique for inhibiting crystal growth of drug nanoparticles in water, and will play a vitally important role in the field of drug nanoparticle preparations, which field will see a fierce competition in development in the future.

## Claims

1. (Currently amended) A method for producing an aqueous dispersion in which nanoparticles including an active ingredient of a pharmaceutical drug are dispersed,
the method comprising the steps of :
(a) preparing (i) a first solution by dissolving a pharmaceutical drug and an ointment base in an organic solvent as a solvent thereof, and (ii) a second solution by dissolving a dispersing agent in water as the solvent of the second solution;
(b) rapidly freezing a liquid mixture of the first solution and the second solution, for crystallization (aggregation of groups of molecules present in a solution) to prepare nanocrystals;
(c) freeze drying a frozen sample of the liquid mixture and
(d) dispersing the freeze-dried sample in water,
wherein the liquid mixture of the first and second solutions is rapidly frozen and freeze-dried so that the active ingredient is coated with the ointment base thereby inhibiting growth of nanocrystals of the active ingredient, and
wherein the ointment base is lanolin, vaseline, beeswax, phenol and zinc oxide liniment, cacao butter, witepsol, glycerogelatin, liquid paraffin, hard fat, macrogol, hydrocarbon gel ointment base, or a lanolin derivative or a mixture of lanolin derivatives which is (i) lauric alcohol, (ii) lauric fatty acid, (iii) a product produced from lanolin through a chemical reaction such as acetylation, alkoxylation, sulfonation, hydrogenation, transesterification, and reduction, such as a metal salt of lauric fatty acid.

2. The method according to claim 1, further comprising the step of
verifying that in a dispersion prepared through the dispersing step, no crystal growth has occurred, until elapse of at least one week in a case where the dispersion has been stored in an environment of 40°C or higher, of nanoparticles containing an active ingredient of the pharmaceutical drug.

3. The method according to claim 2,
wherein
the verifying step is a step of filtering the dispersion prepared through the dispersing step.

4. The method according to claim 2 or 3, further comprising the step of
selecting, from among dispersions prepared through the dispersing step, a dispersion in which no crystal growth has occurred of nanoparticles containing the active ingredient of the pharmaceutical drug.

5. The method according to claim any one of claims 1 to 4,
wherein the liquid mixture is rapidly frozen with liquid nitrogen.

## Patentansprüche

1. Verfahren zur Herstellung einer wässrigen Dispersion, in der Nanopartikel einschließlich eines Wirkstoffs eines pharmazeutischen Medikaments dispergiert sind, wobei das Verfahren die Schritte umfasst:
(a) Herstellen (i) einer ersten Lösung durch Lösen eines pharmazeutischen Medikaments und einer Salbengrundlage in einem organischen Lösungsmittel als ein Lösungsmittel davon und (ii) einer zweiten Lösung durch Lösen eines Dispergiermittels in Wasser als das Lösungsmittel der zweiten Lösung;
(b) schnelles Gefrieren einer flüssigen Mischung der ersten Lösung und der zweiten Lösung zur Kristallisation (Aggregation von Gruppen von Molekülen, die in einer Lösung vorliegen), um Nanokristalle herzustellen;
(c) Gefriertrocknen einer gefrorenen Probe der flüssigen Mischung und
(d) Dispergieren der gefriergetrockneten Probe in Wasser,
wobei die flüssige Mischung der ersten und zweiten Lösungen schnell gefroren und gefriergetrocknet werden, so dass der Wirkstoff mit der Salbengrundlage beschichtet wird, wodurch Wachstum von Nanokristallen des Wirkstoffs inhibiert wird und
wobei die Salbengrundlage Lanolin, Vaseline, Bienenwachs, Phenol und Zinkoxid-Liniment, Kakaobutter, Witepsol, Glycerogelatine, flüssiges Paraffin, Hartfett, Makrogol, Kohlenwasserstoffgel-Salbengrundlage oder ein LanolinDerivat oder eine Mischung von Lanolin-Derivaten ist, das/die (i) Laurinalkohol, (ii) Laurin-Fettsäure, (iii) ein Produkt, das aus Lanolin durch eine chemische Reaktion wie zum Beispiel Acetylierung, Alkoxylierung, Sulfonierung, Hydrierung, Umesterung und Reduktion hergestellt wird, wie zum Beispiel ein Metallsalz von Laurin-Fettsäure.

2. Verfahren nach Anspruch 1, weiterhin umfassend den Schritt
Verifizieren, dass in einer Dispersion, die durch den Dispergierschritt hergestellt wurde, kein Kristallwachstum aufgetreten ist, bis mindestens eine Woche in einem Fall abgelaufen ist, bei dem die Dispersion in einer Umgebung von 40°C oder höher gelagert wurde, von Nanopartikeln, die einen Wirkstoff des pharmazeutischen Medikaments enthalten.

3. Verfahren nach Anspruch 2, wobei der Verifizierungsschritt ein Schritt Filtrieren der Dispersion ist, die durch den Dispergierschritt hergestellt wurde.

4. Verfahren nach Anspruch 2 oder 3, weiterhin umfassend den Schritt Auswählen einer Dispersion, in der kein Kristallwachstum von Nanopartikeln, die den Wirkstoff des pharmazeutischen Medikaments enthalten, aufgetreten ist, aus Dispersionen, die durch den Dispergierschritt hergestellt wurden,.

5. Verfahren nach einem der Ansprüche 1 bis 4, wobei die flüssige Mischung schnell mit flüssigem Stickstoff gefroren wird.

## Revendications

1. Procédé de production d'une dispersion aqueuse dans laquelle des nanoparticules comprenant un principe actif d'un médicament pharmaceutique sont dispersées, le procédé comprenant les étapes suivantes :
(a) préparation (i) d'une première solution par dissolution d'un médicament pharmaceutique et d'une base d'onguent dans un solvant organique en tant que solvant de celle-ci, et (ii) d'une seconde solution par dissolution d'un agent de dispersion dans l'eau en tant que solvant de la seconde solution ;
(b) congélation rapide d'un mélange liquide de la première solution et de la seconde solution, pour la cristallisation (agrégation de groupes de molécules présentes dans une solution) pour préparer des nanocristaux ;
(c) lyophilisation d'un échantillon congelé du mélange liquide et
(d) dispersion de l'échantillon lyophilisé dans de l'eau,
dans lequel le mélange liquide des première et seconde solutions est rapidement congelé et lyophilisé de manière à ce que le principe actif soit revêtu de la base d'onguent inhibant ainsi la croissance des nanocristaux du principe actif, et
dans lequel la base d'onguent est la lanoline, la vaseline, la cire d'abeille, un liniment à base de phénol et d'oxyde de zinc, le beurre de cacao, le witepsol, de la glycérogélatine, de la paraffine liquide, de la graisse dure, un macrogol, une base d'onguent à base de gel hydrocarboné, ou un dérivé de lanoline ou un mélange de dérivés de lanoline, qui est (i) l'alcool laurique, (ii) l'acide gras laurique, (iii) un produit produit à partir lanoline par une réaction chimique telle que l'acétylation, l'alcoxylation, la sulfonation, l'hydrogénation, la transestérification, et la réduction, par exemple un sel métallique d'acide gras laurique.

2. Procédé selon la revendication 1, comprenant en outre l'étape suivante :
vérification que dans une dispersion préparée par l'intermédiaire de l'étape de dispersion, pas de croissance cristalline de nanoparticules contenant un principe actif du médicament pharmaceutique n'a eu lieu, jusqu'à ce qu'au moins une semaine se soit écoulée dans un cas où la dispersion a été stockée dans un environnement à 40 °C ou plus.

3. Procédé selon la revendication 2, dans lequel l'étape de vérification est une étape de filtration de la dispersion préparée par l'intermédiaire de l'étape de dispersion.

4. Procédé selon la revendication 2 ou 3, comprenant en outre l'étape suivante sélection, parmi des dispersions préparées par l'intermédiaire de l'étape de dispersion, d'une dispersion dans laquelle pas de croissance cristalline de nanoparticules contenant le principe actif du médicament pharmaceutique n'a eu lieu.

5. Procédé selon l'une quelconque des revendications 1 à 4, dans lequel le mélange liquide est rapidement congelé avec de l'azote liquide.
